(19) Europäisches Patentamt
European Patent Office
Office européen des brevets

(11) Veröffentlichungsnummer : **0 079 006**
**B1**

# (12) EUROPÄISCHE PATENTSCHRIFT

(45) Veröffentlichungstag der Patentschrift : **11.12.85**

(21) Anmeldenummer : **82109999.1**

(22) Anmeldetag : **29.10.82**

(51) Int. Cl.[4] : **C 07 D233/54**, C 07 D233/60, C 07 D249/08, A 01 N 43/64, A 01 N 43/50

## (54) Azolyl-alkenone und -ole, Verfahren zu ihrer Herstellung sowie ihre Verwendung als Pflanzenwachstumsregulatoren und Fungizide.

(30) Priorität : **10.11.81 DE 3144670**

(43) Veröffentlichungstag der Anmeldung : **18.05.83 Patentblatt 83/20**

(45) Bekanntmachung des Hinweises auf die Patenterteilung : **11.12.85 Patentblatt 85/50**

(84) Benannte Vertragsstaaten : **AT BE CH DE FR GB IT LI NL SE**

(56) Entgegenhaltungen :
**EP-A- 0 016 323**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Elbe, Hans-Ludwig, Dr.**
**Dasnöckel 59**
**D-5600 Wuppertal 11 (DE)**
Erfinder : **Büchel, Karl Heinz, Prof. Dr.**
**Dabringhausener Strasse 42**
**D-5093 Burscheid (DE)**
Erfinder : **Lürssen, Klaus, Dr.**
**August-Kierspel-Strasse 89**
**D-5060 Bergisch-Gladbach (DE)**
Erfinder : **Frohberger, Paul-Ernst, Dr.**
**Willi-Baumeister-Strasse 5**
**D-5090 Leverkusen (DE)**

**EP 0 079 006 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Azolyl-alkenone und -ole, ein Verfahren zu ihrer Herstellung sowie ihre Verwendung als Pflanzenwachstumsregulatoren und Fungizide.

Es ist bereits bekannt geworden, daß bestimmte 6,6-disubstituierte 2,2-Dimethyl-4-(1,2,4-triazol-1-yl)-5-hexen-3-one und -ole gute pflanzenwachstumsregulierende und fungizide Eigenschaften besitzen (vgl. DE-A-2 905 981 und EP-A-0 016 323). Die Wirkung dieser Verbindungen ist jedoch, insbesondere bei niedrigen Aufwandmengen und -konzentrationen, nicht immer voll befriedigend.

Es wurden nun neue Azolyl-alkenone und -ole der Formel

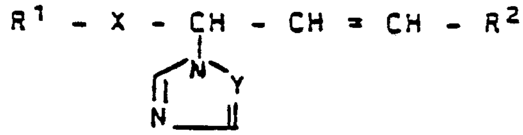

(I)

in welcher $R^1$ für die Gruppierung

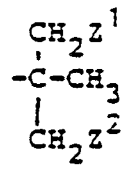

steht, in welcher

$Z^1$ und $Z^2$ gleich oder verschieden sind und für Wasserstoff, Halogen oder Alkyl mit 1 bis 4 Kohlenstoffatomen stehen, und ferner

$R^1$ für Phenyl steht, welches einfach oder mehrfach, gleichartig oder verschieden substituiert sein kann durch Halogen, Alkyl mit 1 bis 4 Kohlenstoffatomen, Alkoxy und Alkylthio mit jeweils 1 bis 4 Kohlenstoffatomen, Alkyl- und Dialkylamino mit jeweils 1 bis 4 Kohlenstoffatomen in jedem Alkylteil, ferner Halogenalkyl, Halogenalkoxy und Halogenalkylthio mit jeweils 1 bis 2 Kohlenstoffatomen und 1 bis 5 gleichen oder verschiedenen Halogenatomen, Phenyl und/oder Phenoxy, wobei die beiden zuletzt genannten Reste ihrerseits durch Halogen und/oder Alkyl mit 1 bis 2 Kohlenstoffatomen substituiert sein können

$R^2$ für geradkettiges oder verzweigtes Alkyl mit 1 bis 12 Kohlenstoffatomen steht, ferner für Phenyl steht, welches einfach oder mehrfach, gleichartig oder verschieden substituiert sein kann durch Halogen, Alkyl mit 1 bis 4 Kohlenstoffatomen, Alkoxy und Alkylthio mit jeweils 1 bis 4 Kohlenstoffatomen, Alkyl- und Dialkylamino mit jeweils 1 bis 4 Kohlenstoffatomen in jedem Alkylteil, ferner Halogenalkyl, Halogenalkoxy und Halogenalkylthio mit jeweils 1 bis 2 Kohlenstoffatomen und 1 bis 5 gleichen oder verschiedenen Halogenatomen, Phenyl und/oder Phenoxy, wobei die beiden zuletzt genannten Reste ihrerseits durch Halogen und/oder Alkyl mit 1 bis 2 Kohlenstoffatomen substituiert sein können, oder

$R^2$ für gegebenenfalls durch Halogen und/oder Alkyl mit 1 bis 4 Kohlenstoffatomen substituiertes Cycloalkyl mit 3 bis 7 Kohlenstoffatomen oder für gegebenenfalls durch Halogen und/oder Alkyl mit 1 bis 4 Kohlenstoffatomen substituiertes Cycloalkylalkyl mit 3 bis 7 Kohlenstoffatomen im Cycloalkylteil und 1 bis 2 Kohlenstoffatomen im Alkylteil steht,

X für die CO- oder CH(OH)-Gruppe steht und

Y für ein Stickstoffatom oder die CH-Gruppe steht,

sowie deren Säureadditionssalze und Metallsalz-Komplexe gefunden.

Die erfindungsgemäßen Verbindungen der Formel (I) kommen in den geometrischen Isomeren E(trans) und Z(cis) vor. Bei der E,Z-Nomenklatur werden die an der Doppelbindung stehenden Substituenten nach der Cahn-Ingold-Prelog-Regel nach abnehmender Priorität eingeordnet. Stehen die bevorzugten Substituenten auf derselben Seite der Doppelbindung, liegt die Konfiguration Z (abgeleitet von zusammen) vor, stehen sie auf entgegengesetzter Seite, liegt die Konfiguration E (abgeleitet von entgegen) vor.

Außerdem besitzen die erfindungsgemäßen Verbindungen der Formel (I) für X=CH(OH) zwei asymmetrische Kohlenstoffatome ; sie können dann in den beiden geometrischen Isomeren (threo- und erythro-Form) vorliegen, die in unterschiedlichen Mengenverhältnissen anfallen können. In beiden Fällen liegen sie als optische Isomere vor.

Weiterhin wurde gefunden, daß man die Azolyl-alkenone und -ole der Formel (I) sowie deren Säureadditions-Salze und Metallsalz-Komplexe erhält, wenn man Verbindungen der Formel

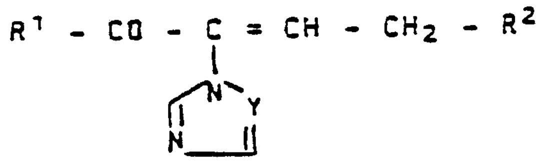

(II)

in welcher $R^1$, $R^2$ und Y die oben angegebene Bedeutung haben, in Gegenwart eines Verdünnungsmittels und gegebenenfalls in Gegenwart eines Katalysators bzw. in Gegenwart von Aluminiumoxid erhitzt und gegebenenfalls die dabei erhaltenen erfindungsgemäßen Azolyl-alkenone der Formel

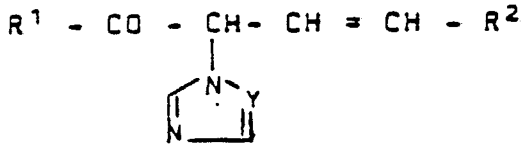

(Ia)

in welcher $R^1$, $R^2$ und Y die oben angegebene Bedeutung haben, in allgemein üblicher Weise reduziert und gegebenenfalls anschließend an die so erhaltenen Verbindungen der Formel (I) eine Säure oder ein Metallsalz addiert.

Schließlich wurde gefunden, daß die neuen Azolyl-alkenone und -ole der Formel (I) sowie deren Säureadditions-Salze und Metallsalz-Komplexe starke pflanzenwachstumsregulierende und starke fungizide Eigenschaften aufweisen.

Die erfindungsgemäßen Wirkstoffe sind unter anderem dadurch charakterisiert, daß sie an dem endständigen Kohlenstoffatom der Alkylgruppe ein Wasserstoffatom sowie einen weiteren von Wasserstoff verschiedenen Substituenten enthalten. Sie unterscheiden sich dadurch eindeutig von den aus der EP-A-0 016 323 bekannten Verbindungen, in denen das endständige Kohlenstoffatom der Allyl-Einheit jeweils zwei von Wasserstoff verschiedene Reste trägt.

Überraschenderweise besitzen die erfindungsgemäßen Stoffe eine bessere pflanzenwachstumsregulierende und fungizide Wirkung als die in der EP-A-0 016 323 offenbarten 6,6-disubstituierten 2,2-Dimethyl-4-(1,2,4-triazol-1-yl)-5-hexan-3-one und -ole, welche die chemisch und wirkungsmäßig naheliegendsten vorbekannten Verbindungen sind.

Besonders bevorzugt sind diejenigen Verbindungen der Formel (I), in denen $R^1$ für die Gruppierung

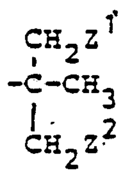

steht, wobei

$Z^1$ und $Z^2$ gleich oder verschieden sind und für Wasserstoff, Fluor, Chlor, Brom, Methyl, Ethyl, Propyl oder Butyl stehen ; sowie für gegebenenfalls einfach bis dreifach, gleich oder verschieden substituiertes Phenyl steht, wobei als Substituenten genannt seien : Fluor, Chlor, Methyl, Isopropyl, tert.-Butyl, Methoxy, Methylthio, Isopropoxy, Trifluormethyl, Trifluormethoxy, Trifluormethylthio, Methylamino, Dimethylamino, und gegebenenfalls durch Fluor, Chlor oder Methyl substituiertes Phenyl und Phenoxy ;

$R^2$ für geradkettiges oder verzweigtes Alkyl mit 1 bis 8 Kohlenstoffatomen steht ; ferner für gegebenenfalls einfach bis dreifach, gleich oder verschieden substituiertes Phenyl steht, wobei als Substituenten genannt seien : Fluor, Chlor, Methyl, Isopropyl, tert.-Butyl, Methoxy, Methylthio, Isopropoxy, Trifluormethyl, Trifluormethoxy, Trifluormethylthio, Methylamino, Dimethylamino, und gegebenenfalls durch Fluor, Chlor oder Methyl substituiertes Phenyl und Phenoxy ; und

$R^2$ weiterhin für jeweils gegebenenfalls durch Methyl, Ethyl, Isopropyl, Fluor oder Chlor substituiertes Cyclopropyl, Cyclobutyl, Cyclopentyl, Cyclohexyl, Cyclopropylmethyl, Cyclobutylmethyl, Cyclopentylmethyl, Cyclohexylmethyl steht ;

X für die CO- oder CH(OH)-Gruppe steht und

Y für ein Stickstoffatom oder die CH-Gruppe steht.

Im einzelnen seien außer den bei den Herstellungsbeispielen genannten Verbindungen folgende Verbindungen der Formel (I) genannt (wobei X für die CO- oder die CH(OH)-Gruppe steht und Y für ein Stickstoffatom oder die CH-Gruppe steht) :

(Siehe Tabelle 1 Seite 4 ff.)

Tabelle 1

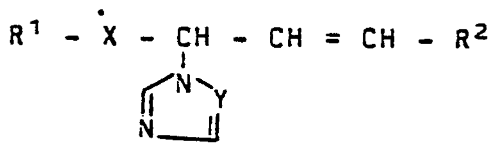 (I)

| $R^1$ | $R^2$ |
|---|---|
| 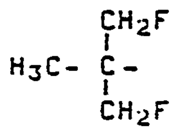 | $-CH_2-CH(CH_3)_2$ |
| 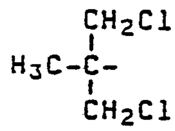 | $-CH_2-CH(CH_3)_2$ |
| $(CH_3)_3C-$ | 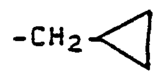 |
| $(CH_3)_3C-$ | 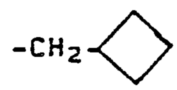 |
| $(CH_3)_3C-$ | 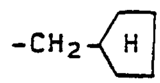 |
| $(CH_3)_3C-$ | 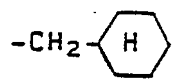 |
| $(CH_3)_3C-$ | $-CH_2-CH(CH_3)C_2H_5$ |
| $(CH_3)_3C-$ | $-CH_2-CH(CH_3)C_3H_7$ |
| $(CH_3)_3C-$ | $-CH_2-CH(CH_3)C_4H_9$ |
| $(CH_3)_3C-$ | $-CH_2-C(CH_3)_3$ |
| $(CH_3)_3C-$ | $-CH_2-CH(C_2H_5)_2$ |
| $(CH_3)_3C-$ | $-CH_2-CH(C_3H_7)_2$ |
| $(CH_3)_3C-$ | 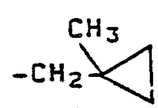 |
| $(CH_3)_3C-$ | 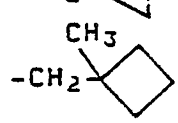 |
| $(CH_3)_3C-$ | 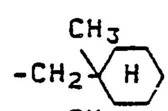 |
| $(CH_3)_3C-$ | 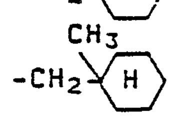 |
| $(CH_3)_3C-$ | $-CH(CH_3)-CH(CH_3)_2$ |
| $(CH_3)_3C-$ | $-C(CH_3)_2-CH(CH_3)_2$ |

Tabelle 1 (Fortsetzung)

| $R^1$ | $R^2$ |
|---|---|
| $(CH_3)_3C-$ | 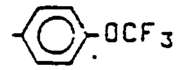 |
| $(CH_3)_3C-$ | 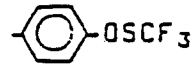 |
| $(CH_3)_3C-$ | 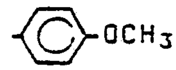 |
| $(CH_3)_3C-$ | 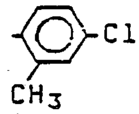 |
| $(CH_3)_3C-$ | 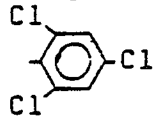 |
| $(CH_3)_3C-$ | 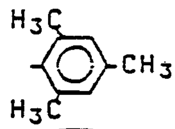 |
| $(CH_3)_3C-$ | 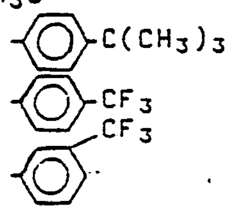 |
| $(CH_3)_3C-$ | |
| $(CH_3)_3C-$ | |
| $(CH_3)_3C-$ | 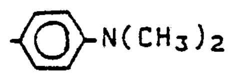 |
| $(CH_3)_3C-$ | 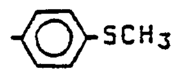 |
| $(CH_3)_3C-$ | 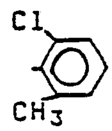 |
| $(CH_3)_3C-$ | 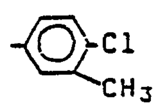 |
| $(CH_3)_3C-$ | 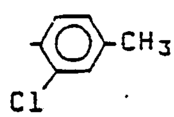 |

Bevorzugte erfindungsgemäße Verbindungen sind auch Additionsprodukte aus Säuren und denjenigen Azolyl-alkenonen und -olen der Formel (I), in denen $R^1$, $R^2$, X und Y die Bedeutung haben, die bereits als besonders bevorzugt für diese Reste genannt wurden.

Zu den Säuren, die addiert werden können, gehören vorzugsweise Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Citronensäure, Salizylsäure, Sorbinsäure und Milchsäure, sowie Sulfonsäuren, wie z. B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen Azolyl-alkenonen und -olen der Formel (I), in denen $R^1$, $R^2$, X und Y die Bedeutungen haben, die bereits als besonders bevorzugt für diese Reste genannt wurden.

Hierbei sind Salze der Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z. B. die Chlorwasserstoffsäure und die Bromwasserstoffsäure, ferner Phosphorsäure, Salpetersäure und Schwefelsäure.

Verwendet man beispielsweise 6-Cyclohexyl-2,2-dimethyl-4-(1,2,4-triazol-1-yl)-4-hexen-3-on als Ausgangsstoff, Aluminiumoxid als Reaktionskomponente und Methanol als Verdünnungsmittel, so kann der Reaktionsablauf nach dem erfindungsgemäßen Verfahren durch das folgende Formelschema wiedergegeben werden :

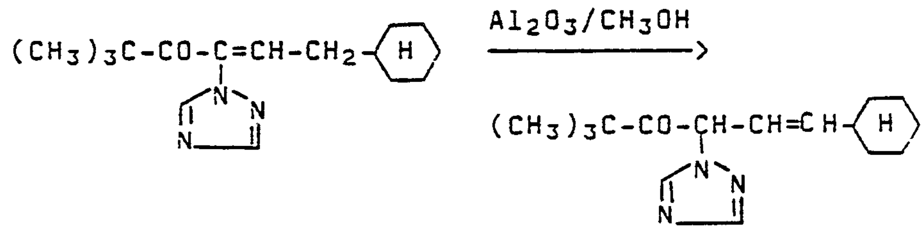


Verwendet man beispielsweise 6-Cyclohexyl-2,2-dimethyl-4-(1,2,4-triazol-1-yl)-5-hexen-3-on als Ausgangsstoff und Natriumborhydrid als Reduktionsmittel, so kann der Reaktionsablauf nach dem erfindungsgemäßen Verfahren durch das folgende Formelschema wiedergegeben werden :

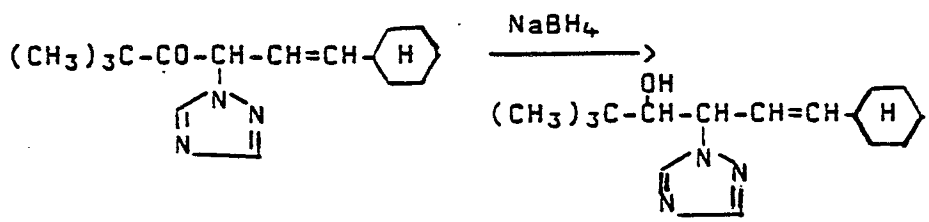


Die Verbindungen der Formel (II) sind bekannt oder lassen sich in einfacher Weise nach im Prinzip bekannten Verfahren herstellen (vgl. DE-A-3 000 643 sowie die dort zitierte Literatur). So erhält man Verbindungen der Formel (II), indem man Ketoenamine der Formel

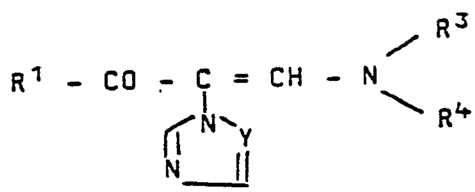 (III)

in welcher

$R^1$ und Y die oben angegebene Bedeutung haben und

$R^3$ und $R^4$ gleich oder verschieden sind und für Alkyl mit 1 bis 4 Kohlenstoffatomen, insbesondere Methyl, stehen,

mit magnesium-organischen Verbindungen der Formel

$$Hal—Mg—R^2 \qquad (IV)$$

in welcher

$R^2$ die oben angegebene Bedeutung hat und

Hal für Halogen steht,

in Gegenwart eines inerten organischen Lösungsmittels, wie beispielsweise Ether, und gegebenenfalls in Gegenwart eines Inertgases, wie beispielsweise Stickstoff, bei Temperaturen zwischen − 20 und 120 °C umsetzt (vgl. hierzu auch DE-A-3 000 643 sowie die Herstellungsbeispiele).

Die Ketoenamine der Formel (III) sind bekannt oder lassen sich nach im Prinzip bekannten Verfahren in einfacher Weise herstellen (vgl. DE-A-3 000 643). So erhält man Ketoenamine der Formel (III), indem man Azolylketone der Formel

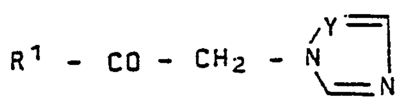 (V)

in welcher $R^1$ und Y die oben angegebene Bedeutung haben, mit Amidacetalen bzw. Aminalestern der Formeln

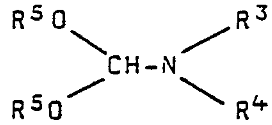

(VIa)

bzw.

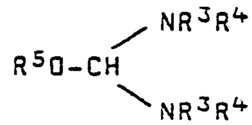

(VIb)

in welchen

$R^3$ und $R^4$ die oben angegebene Bedeutung haben und

$R^5$ für Alkyl mit 1 bis 4 Kohlenstoffatomen steht,

in an sich bekannter Art und Weise in Gegenwart eines inerten organischen Lösungsmittels, wie beispielsweise eines aromatischen Kohlenwasserstoffes und wie insbesondere eines im Überschuß eingesetzten Amidacetals bzw. Aminalesters der Formel (VIa) bzw. (VIb) in der Siedehitze umsetzt (vergleiche hierzu auch Chem. Ber. *101*, 41-50 (1968) ; J. Org. Chem. *43*, 4248-50 (1978) sowie die Herstellungsbeispiele).

Die Azolyl-ketone der Formel (V) sind bekannt (vergleiche z. B. DE-A-2 431 407, DE-A-2 610 022 und DE-A-2 638 470) ; bzw. lassen sie sich nach üblichen Methoden herstellen, indem man die entsprechenden Halogen-ketone in Gegenwart eines Säurebinders mit 1,2,4-Triazol oder Imidazol umsetzt.

Die Amidacetale bzw. Aminalester der Formeln (VIa) bzw. (VIb) sind allgemein bekannte Verbindungen der organischen Chemie (vergleiche z. B. Chem. Ber. *101*, 41-50 (1968) und J. Org. Chem. *43*, 4248-50 (1978)).

Die magnesium-organischen Verbindungen der Formel (IV) sind allgemein bekannte Verbindungen der organischen Chemie.

Als Verdünnungsmittel kommen für das erfindungsgemäße Verfahren zur Herstellung der Azolyl-alkenone der Formel (Ia) unter den Reaktionsbedingungen inerte organische Lösungsmittel in Frage. Hierzu gehören vorzugsweise Ketone, wie Aceton und Methylethylketon ; Alkohole, wie Methanol, Ethanol oder Isopropanol ; aliphatische und aromatische Kohlenwasserstoffe, wie Benzol, Toluol oder Xylol ; sowie halogenierte Kohlenwasserstoffe, wie Methylenchlorid, Tetrachlorkohlenstoff, Chloroform oder Chlorbenzol.

Das erfindungsgemäße Verfahren zur Herstellung der Azolyl-alkenone der Formel (Ia) wird gegebenenfalls in Gegenwart einer Base als Katalysator durchgeführt. Hierzu gehören vorzugsweise organische Stickstoffbasen, wie Morpholin, Pyridin, Triethylamin und N,N-Dimethylbenzylamin.

Die Reaktionstemperaturen können bei dem erfindungsgemäßen Verfahren zur Herstellung der Azolyl-alkenone der Formel (Ia) in einem größeren Bereich variiert werden. Im allgemeinen arbeitet man bei Temperaturen zwischen 30 und 150 °C, vorzugsweise zwischen 50 und 120 °C.

Die Durchführung des erfindungsgemäßen Verfahrens zur Herstellung der Azolyl-alkenone der Formel (Ia) erfolgt entweder rein thermisch durch Erhitzen der Verbindungen der Formel (II), oder basenkatalytisch, wobei auf 1 Mol der Verbindungen der Formel (II) 0,1 bis 1 Mol an Base eingesetzt wird, oder in Gegenwart von Aluminiumoxid. Die Isolierung der erfindungsgemäßen Verbindungen erfolgt in allen Fällen in üblicher Art und Weise.

Die erfindungsgemäße Reduktion zur Herstellung der Azolyl-alkenole der Formel (I) erfolgt in üblicher Art und Weise, z. B. durch Umsetzung von Azolyl-alkenonen der Formel (Ia) mit komplexen Hydriden, gegebenenfalls in Gegenwart eines Verdünnungsmittels, oder durch Umsetzung von Azolyl-alkenonen der Formel (Ia) mit Aluminiumisopropylat in Gegenwart eines Verdünnungsmittels.

Arbeitet man mit komplexen Hydriden, so kommen als Verdünnungsmittel für diese erfindungsgemäße Umsetzung polare organische Lösungsmittel infrage. Hierzu gehören vorzugsweise Alkohole, wie Methanol, Ethanol, Butanol, Isopropanol und Ether, wie Diethylether oder Tetrahydrofuran. Die Reaktion wird im allgemeinen bei 0 bis 30 °C, vorzugsweise bei 0 bis 20 °C durchgeführt. Hierzu setzt man auf 1 Mol des Ketons der Formel (Ia) etwa 1 Reaktionsäquivalent eines komplexen Hydrids, wie Natriumborhydrid oder Lithiumalanat, ein. Zur Isolierung der reduzierten Verbindungen der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für diese erfindungsgemäße Umsetzung bevorzugt Alkohole, wie Isopropanol, oder inerte Kohlenwasserstoffe, wie Benzol, infrage. Die Reaktionstemperaturen können wiederum in einen größeren Bereich variiert werden ; im allgemeinen arbeitet man zwischen 20 und 120 °C, vorzugsweise bei 50 bis 100 °C. Zur Durchführung der Reaktion setzt man auf 1 Mol des Ketons der Formel (Ia) etwa 1 bis 2 Mol Aluminiumisopropylat ein. Zur Isolierung der reduzierten Verbindungen der Formel (I) wird das überschüssige Lösungsmittel durch

Destillation im Vakuum entfernt und die entstandene Aluminiumverbindung mit verdünnter Schwefelsäure oder Natronlauge zersetzt. Die weitere Aufarbeitung erfolgt in üblicher Weise.

Zur Herstellung von Säureadditions-Salzen der Azolylalkenone und -ole der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen von Azolyl-alkenonen und -olen der Formel (I) kommen vorzugsweise Salze von denjenigen Anionen und Kationen in Betracht, die bereits im Zusammenhang mit der Beschreibung der erfindungsgemäßen Metallsalz-Komplexe als bevorzugt genannt wurden.

Die Metallsalz-Komplexe von Verbindungen der Formel (I) können in einfacher Weise nach üblichen Verfahren erhalten werden, so z. B. durch Lösen des Metallsalzes in Alkohol, z. B. Ethanol, und Hinzufügen zur Verbindung der Formel (I). Man kann Metallsalz-Komplexe in bekannter Weise, z. B. durch Abfiltrieren, isolieren und gegebenenfalls durch Umkristallisation reinigen.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens (« Lagerns ») der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, sodaß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragsteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, sodaß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z. B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein

kann. Andererseits ist es aber auch möglich, das Wachstum der Seitentriebe zu hemmen. Für diese Wirkung besteht z. B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z. B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden (« Ausdünnung »), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüberhinaus kann mit Wachstumsregulatoren auch eine zeitliche Konzentrierung der Reife erzielt werden. Damit werden die Voraussetzungen dafür geschaffen, daß z. B. bei Tabak, Tomaten und Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, sodaß die Pflanzen, wie z. B. Ananas oder Zierpflanzen in Gärtnereien, zu einem Zeitpunkt keimen, austreiben und blühen, an dem sie normalerweise hierzu keine Bereitschaft zeigen. Eine Verzögerung des Austreibens von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die erfindungsgemäßen Wirkstoffe weisen auch eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen, so zur Bekämpfung von Erysiphe-Arten, wie z. B. gegen den Erreger des Gersten- bzw. des Getreidemehltaus (Erysiphe graminis), zur Bekämpfung der Streifenkrankheit der Gerste (Drechslera graminea), zur Bekämpfung von Venturia-Arten, wie z. B. gegen den Erreger des Apfelschorfs (Venturia inaequalis), oder zur Bekämpfung von Reiskrankheiten, wie z. B. Pyricularia oryzae und Pellicularia sasakii.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid. Als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage : z. B. gebrochene und fraktionierte natürliche

Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage : z. B. nichtionogene und anionische Emulgatoren, wie Polyoxyethylen-Fettsäureester, Polyoxyethylen-Fettalkoholether, z. B. Alkylarylpolyglycol-ether, Alkylsulfonate, Alkylsulfate, Arylsulfonate sowie Eiweißhydrolysate. Als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z. B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Auch beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 Gew.-%. Bei der Saatgutbehandlung werden im allgemeinen Wirkstoffmengen von 0,001 bis 50 g je kg Saatgut, vorzugsweise 0,01 bis 10 g, benötigt. Bei Behandlung des Bodens sind Wirkstoffkonzentrationen von 0,000 01 bis 0,1 Gew.-%, vorzugsweise von 0,000 1 bis 0,02 %, am Wirkungsort erforderlich.

Die Herstellung und die Verwendung der erfindungsgemäßen Stoffe werden durch die folgenden Beispiele veranschaulicht.

## Herstellungsbeispiele

### Beispiel 1

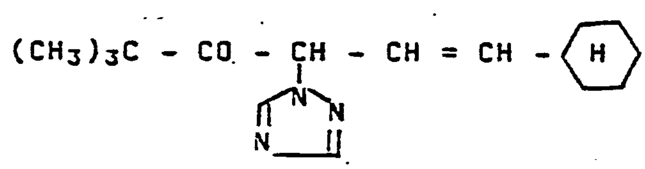

20 g (0,073 Mol) 6-Cyclohexyl-2,2-dimethyl-4-(1,2,4-triazol-1-yl)-4-hexen-3-on und 200 g Aluminiumoxid werden in 300 ml Methanol 24 Stunden unter Rückfluß erhitzt. Man läßt das Reaktionsgemisch abkühlen, saugt über Kieselgur ab und engt das Filtrat ein. Man erhält 19,9 g (99 % der Theorie) 6-Cyclohexyl-2,2-dimethyl-4-(1,2,4-triazol-1-yl)-5-hexen-3-on vom Brechungsindex $n_D^{20} = 1,489\,0$.

### Herstellung des Ausgangsproduktes

a)

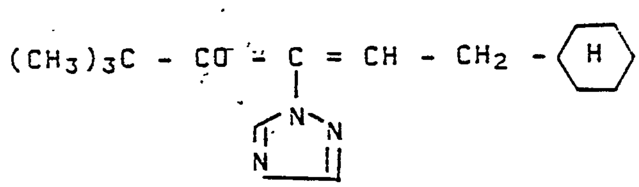

44,4 g (0,2 Mol) 2,2-Dimethyl-5-dimethylamino-4-(1,2,4-triazol-1-yl)-4-penten-3-on werden in 600 ml Ether gelöst und bei – 20 °C mit einer Lösung von 48,2 g (0,24 Mol) Cyclohexylmethyl-magnesiumbromid in 200 ml Ether versetzt. Man läßt 1,5 Stunden nachrühren, wobei sich das Reaktionsgemisch auf Raumtemperatur erwärmt. Mit verdünnter Salzsäure wird das Reaktionsgemisch auf einen pH-Wert von 7 bis 8 eingestellt. Danach wird die organische Phase abgetrennt, zweimal mit Wasser gewaschen, über Natriumsulfat getrocknet und eingeengt. Man erhält 27,4 g (49,8 % der Theorie) 6-Cyclohexyl-2,2-dimethyl-4-(1,2,4-triazol-1-yl)-4-hexen-3-on vom Brechungsindex $n_D^{20}$ = 1,491 0.

b) 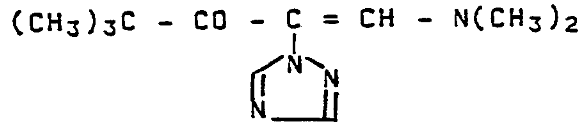

250,8 g (1,5 Mol) 2,2-Dimethyl-4-(1,2,4-triazol-1-yl)-3-butanon werden mit 196 g (1,65 Mol) Dimethylformamid-dimethylacetal 5 Stunden unter Rückfluß erhitzt. Danach wird das überschüssige Acetal abdestilliert. Man erhält 306 g (92 % der Theorie) 2,2-Dimethyl-5-dimethylamino-4-(1,2,4-triazol-1-yl)-4-penten-3-on vom Brechungsindex $n_D^{20}$ = 1,531.

c) 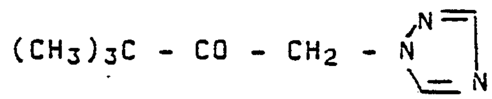

138 g (2 Mol) 1,2,4-Triazol werden bei Raumtemperatur portionsweise zu 276,4 g (2 Mol) gemahlenem Kaliumcarbonat und 296,2 g (2 Mol) α-Chlorpinakolin in 500 ml Aceton gegeben, wobei die Innentemperatur bis zur Siedehitze ansteigt. Man läßt 5 Stunden unter Rückfluß rühren und kühlt dann auf Raumtemperatur ab. Das Reaktionsgemisch wird filtriert und das Filtrat durch Abdestillieren des Lösungsmittels im Vakuum eingeengt. Der ölige Rückstand kristallisiert nach Zugabe von Benzin. Man erhält 240,8 g (72 % der Theorie) 2,2-Dimethyl-4-(1,2,4-triazol-1-yl)-3-butanon vom Schmelzpunkt 62-64 °C.

Beispiel 2

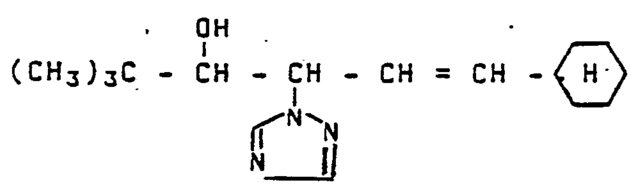

10,4 g (0,038 Mol) 6-Cyclohexyl-2,2-dimethyl-4-(1,2,4-triazol-1-yl)-5-hexen-3-on (Beispiel 1) werden in 100 ml Methanol gelöst und bei – 10 °C tropfenweise mit einer Lösung von 0,38 g (0,01 Mol) Natriumborhydrid in 5 ml Eiswasser versetzt. Man läßt 2 Stunden nachrühren und stellt dann das Reaktionsgemisch mit verdünnter Salzsäure auf einen pH-Wert von 6 bis 7. Das Reaktionsgemisch wird durch Abdestillieren des Lösungsmittels im Vakuum eingeengt. Der Rückstand wird in Methylenchlorid aufgenommen, mit Wasser gewaschen, über Natriumsulfat getrocknet und eingeengt. Man erhält 8,9 g (86 % der Theorie) 6-Cyclohexyl-2,2-dimethyl-4-(1,2,4-triazol-1-yl)-5-hexen-3-ol vom Brechungsindex $n_D^{20}$ = 1,492 0.

In entsprechender Weise und gemäß den angegebenen Verfahren werden die folgenden Verbindungen der Formel (I) erhalten :

Tabelle 2

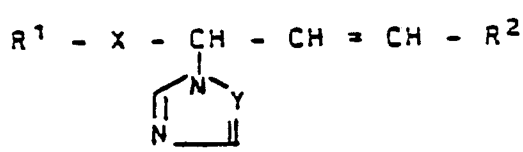 (I)

| Bsp. Nr. | $R^1$ | X | Y | $R^2$ | Schmelzpunkt(°C), Brechungsindex $n_D^{20}$ |
|---|---|---|---|---|---|
| 3 | $(CH_3)_3C-$ | CO | N | $-C_6H_{13}$ | 1,5388 |
| 4 | $(CH_3)_3C-$ | CO | N | $-C_7H_{15}$ | 1,4778 |

Tabelle 2 (Fortsetzung)

| Bsp. Nr. | $R^1$ | X | Y | $R^2$ | Schmelzpunkt(°C) Brechungsindex $n_D^{20}$ |
|---|---|---|---|---|---|
| 5 | $(CH_3)_3C-$ | CO | N | $-C_3H_7$ | 1,4880 |
| 6 | $(CH_3)_3C-$ | CO | N | 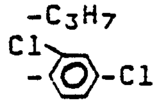 | 105-08 |
| 7 | $(CH_3)_3C-$ | CO | N | 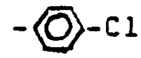 | 1,5512 |
| 8 | $(CH_3)_3C-$ | CO | N | $-C_3H_7-i$ | 1,4871 |
| 9 | $(CH_3)_3C-$ | CO | N | $-CH_2-C_3H_7-i$ | 1,4868 |
| 10 | $(CH_3)_3C-$ | CO | N | 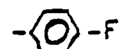 | 1,5434 |
| 11 | $(CH_3)_3C-$ | CO | N | 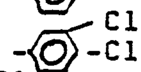 | 138-40 |
| 12 | $(CH_3)_3C-$ | CO | N | 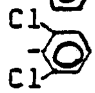 | 1,5624 |
| 13 | $(CH_3)_3C-$ | CO | N | $-CH(C_2H_5)_2$ | 1,4885 |
| 14 | $ClCH_2C(CH_3)_2-$ | CO | N | $-CH_2-C_3H_7-i$ | 1,4969 |
| 15 | $FCH_2C(CH_3)_2-$ | CO | N | $-CH_2-C_3H_7-i$ | 1,4777 |
| 16 | $(CH_3)_3C-$ | CH(OH) | N | 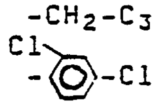 | zähes Oel |
| 17 | $(CH_3)_3C-$ | CH(OH) | N | $-C_3H_7$ | 1,4898 |
| 18 | $(CH_3)_3C-$ | CH(OH) | N | $-C_6H_{13}$ | 1,4798 |
| 19 | $(CH_3)_3C-$ | CH(OH) | N | 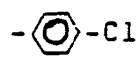 | 1,5633 |
| 20 | $(CH_3)_3C-$ | CH(OH) | N | $-C_3H_7-i$ | 1,4841 |
| 21 | $(CH_3)_3C-$ | CH(OH) | N | $-CH_2-C_3H_7-i$ | 1,4857 |
| 22 | $(CH_3)_3C-$ | CH(OH) | N | $-C_7H_{15}$ | 1,4807 |
| 23 | $(CH_3)_3C-$ | CH(OH) | N | 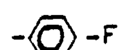 | 112-24 |
| 24 | $(CH_3)_3C-$ | CH(OH) | N | 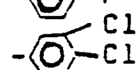 | 154-56(A-Form) |
| 25 | $(CH_3)_3C-$ | CH(OH) | N | 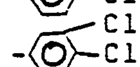 | 46-48(B-Form) |
| 26 | $(CH_3)_3C-$ | CH(OH) | N | 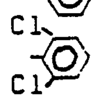 | 180 (A-Form) |
| 27 | $(CH_3)_3C-$ | CH(OH) | N | $-CH(C_2H_5)C_4H_9$ | 1,4832 |
| 28 | $(CH_3)_3C-$ | CH(OH) | N | $-CH(C_2H_5)C_4H_9$ | 1,4832 |
| 29 | $ClCH_2C(CH_3)_2-$ | CH(OH) | N | $-CH_2-C_3H_7-i$ | 1,5001 |
| 30 | $FCH_2C(CH_3)_2-$ | CH(OH) | N | $-CH_2-C_3H_7-i$ | 1,4852 |
| 31 | 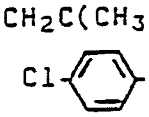 | CH(OH) | N | 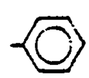 | 50-60 |

A- und B-Form = die beiden möglichen geometrischen Isomeren

Tabelle 2 (Fortsetzung)

| Bsp. Nr. | $R^1$ | X | Y | $R^2$ | Schmelzpunkt (°C) Brechungsindex $n_D^{20}$ |
|---|---|---|---|---|---|
| 32 | $(CH_3)_3C-$ | CO | CH | $-C_3H_7$ | 1,4915 |
| 33 | $(CH_3)_3C-$ | CO | CH | $-C_6H_{13}$ | 1,4773 |
| 34 | $(CH_3)_3C-$ | CO | CH | $-C_7H_{13}$ | 1,4808 |
| 35 | $(CH_3)_3C-$ | CO | CH | $-C_8H_{17}$ | 1,4742 |
| 36 | $(CH_3)_3C-$ | CO | CH | $-C_9H_{19}$ | 1,4770 |
| 37 | $(CH_3)_3C-$ | CO | CH | $-C_5H_{11}$ | 1,4890 |
| 38 | $(CH_3)_3C-$ | CO | CH | 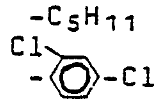 | zähes Oel |
| 39 | $(CH_3)_3C-$ | CO | CH | 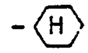 | 1,5018 |
| 40 | $(CH_3)_3C-$ | CO | CH | $-C_5H_{11}$ | 1,4892 |
| 41 | $(CH_3)_3C-$ | CH(OH) | CH | 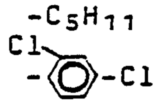 | 92-96 |
| 42 | $(CH_3)_3C-$ | CH(OH) | CH | $-C_6H_{13}$ | 1,4742 |
| 43 | $(CH_3)_3C-$ | CH(OH) | CH | 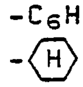 | 1,5050 |
| 44 | 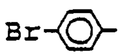 | CO | N | 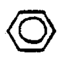 | 46 |
| 45 | 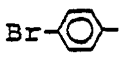 | CO | N | 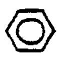 | 107-11 (x $CuCl_2$) |
| 46 | $C_3H_7-C(CH_3)_2-$ | CH(CH) | N | $-CH_2-C_3H_7$-i | 1,4849 |
| 47 | 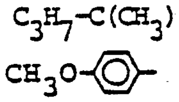 | CH(OH) | N | $C_2H_5$ | 1,5320 (A-Form) |
| 48 | $(CH_3)_3C-$ | CO | N | $-CH_2-C(CH_3)_3$ | 1,4828 |
| 49 | $(CH_3)_3C-$ | CO | N | $-CH_2-$ 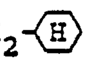 | 1,4884 |
| 50 | $(CH_3)_3C-$ | CH(OH) | N | $-CH_2-$ 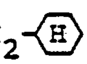 | 1,4887 |
| 51 | $(CH_3)_3C-$ | CH(OH) | N | $-CH_2-C(CH_3)_3$ | 62 |
| 52 | 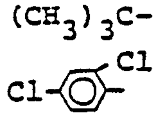 | CH(OH) | N | $-CH_2-C_3H_7$-i | 49 |
| 53 | 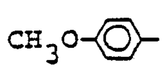 | CH(OH) | N | $C_2H_5$ | 40 (B-Form) |
| 54 | 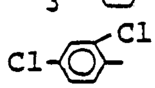 | CH(OH) | N | $C_2H_5$ | 67 |
| 55 | $C_3H_7-C(CH_3)_2-$ | CO | N | $-CH_2-C_3H_7$-i | 1,4819 |
| 56 | $(CH_3)_3C-$ | CO | N | $-CH_2-CH(CH_3)-C_2H_5$ | 1,4772 |
| 57 | $(CH_3)_3C-$ | CH(OH) | N | $-CH_2-CH(CH_3)-C_2H_5$ | 1,4819 |
| 58 | 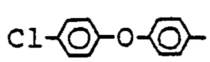 | CO | N | $CH_3$ | 1,5996 |

A- und B-Form = die beiden möglichen geometrischen Isomeren

## Verwendungsbeispiele

In den folgenden Beispielen werden die nachstehend angegebenen Verbindungen als Vergleichssubstanzen eingesetzt :

(A) = 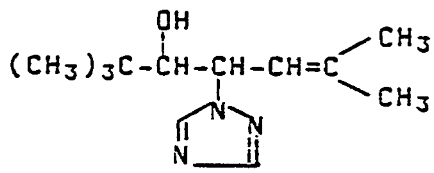 (EP-A 0 016 323 ; Beispiel 24)

(B) = 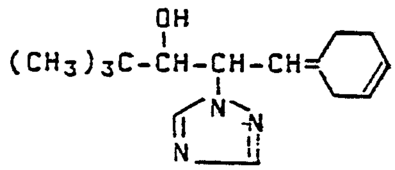 (EP-A 0 016 323 ; Beispiel 13)

(C) = 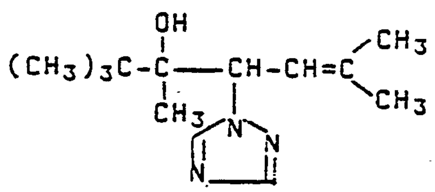 (EP-A 0 016 323 ; Beispiel 3)

(D) = 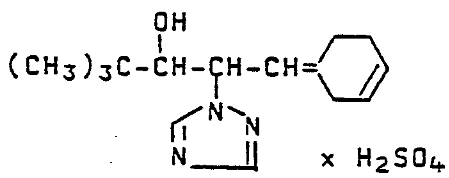 (EP-A 0 016 323 ; Beispiel 15)

(E) = 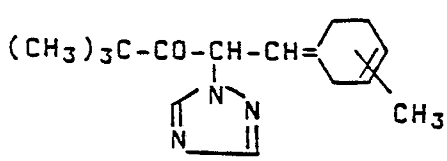 (EP-A 0 016 323 ; Beispiel 9)

### Beispiel A

Wuchshemmung bei Sojabohnen

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Die erfindungsgemäßen Wirkstoffe 21 und 19 zeigen in diesem Test eine bessere Wuchshemmung als die aus dem Stand der Technik bekannte Verbindung (A).

### Beispiel B

Wuchshemmung bei Gras (Festuca pratensis)

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gras (Festuca pratensis) wird im Gewächshaus bis zu einer Wuchshöhe von 5 cm angezogen. In diesem Stadium werden die Pflanzen mit den Wirkstoffzubereitungen tropfnaß besprüht. Nach 3 Wochen wird der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstums entsprechend dem der Kontrollpflanzen.

Der erfindungsgemäße Wirkstoff 21 zeigt in diesem Test eine wesentlich bessere Wuchshemmung als die aus dem Stand der Technik bekannten Verbindungen (A) und (B).

## Beispiel C

Wuchsbeeinflussung bei Zuckerrüben

Lösungsmittel : 30 Gewichtsteile Dimethylformamid
Emulgator : 1 Gewichtsteile Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit der Wirkstoffzubereitung besprüht. Nach 14 Tagen wird der Zuwachs der Pflanzen gemessen und die Wuchsbeeinflussung in Prozent des Zuwachses der Kontrollpflanzen berechnet. Es bedeuten 0 % Wuchsbeeinflussung ein Wachstum entsprechend dem der Kontrollpflanzen. Negative Werte kennzeichnen eine Wuchshemmung, positive eine Wuchsförderung gegenüber den Kontrollpflanzen.

Die erfindungsgemäßen Wirkstoffe 16, 18, 19, 21, 26, 36 und 41 zeigen in diesem Test eine stärkere Wuchsbeeinflussung als die aus dem Stand der Technik bekannten Verbindungen (C) und (D).

## Beispiel D

Wuchshemmung bei Baumwolle

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf. Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 3 Wochen wird der Zuwachs der Pflanzen gemessen und die Wuchshemmung in Prozent des Zuwachses der Kontrolle berechnet. Es bedeuten 100 % Wuchshemmung den Stillstand des Wachstums und 0 % ein Wachstum entsprechend dem der Kontrollpflanzen.

Die erfindungsgemäßen Wirkstoffe 1, 6, 10, 11, 16 und 25 zeigen in diesem Test eine bessere Wuchshemmung als die aus dem Stand der Technik bekannten Verbindungen (A), (B) und (E).

## Beispiel E

Stimulierung der $CO_2$-Fixierung bei Sojabohnen

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Im weiteren Verlauf des Experimentes wird die $CO_2$-Fixierung der Pflanzen mit üblichen Methoden gemessen. Die Werte werden mit denen nicht mit Wirkstoffen behandelter Kontrollpflanzen vergleichen.

Es bedeuten :
— Hemmung der $CO_2$-Fixierung
O $CO_2$-Fixierung wie bei Kontrolle
+ geringe Stimulierung der $CO_2$-Fixierung
++ starke Stimulierung der $CO_2$-Fixierung
+++ sehr starke Stimulierung der $CO_2$-Fixierung

Die erfindungsgemäßen Wirkstoffe 1 und 22 zeigen in diesem Test eine Stimulierung der $CO_2$-Fixierung im Gegensatz zu den aus dem Stand der Technik bekannten Verbindungen (A), (B) und (E).

### Beispiel F

Erysiphe-Test (Gerste)/protektiv

Lösungsmittel : 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f. sp. hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber der aus dem Stand der Technik bekannten Verbindung (E) zeigen bei diesem Test z. B. die Verbindungen gemäß folgender Herstellungsbeispielen : 5, 32, 34, 35, 36, 40, 42, 38, 6, 17, 18, 19, 20, 21, 22, 10, 11, 25 und 12.

### Beispiel G

Drechslera graminea-Test (Gerste)/Saatgutbehandlung (syn. Helminthosporium gramineum)

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Das Saatgut setzt man in gesiebter, feuchter Standarderde eingebettet, in verschlossenen Petrischalen im Kühlschrank 10 Tage lang einer Temperatur von 4 °C aus. Dabei wird die Keimung der Gerste und gegebenenfalls auch der Pilzsporen eingeleitet. Anschließend sät man die vorgekeimte Gerste mit 2 × 50 Korn 3 cm tief in eine Standarderde und kultiviert sie im Gewächshaus bei einer Temperatur von ca. 18 °C in Saatkästen, die täglich 15 Stunden dem Licht ausgesetzt werden.

Ca. 3 Wochen nach der Aussaat erfolgt die Auswertung der Pflanzen auf Symptome der Streifenkrankheit.

Eine deutliche Überlegenheit in der Wirksamkeit gegenüber der aus dem Stand der Technik bekannten Verbindung (B) zeigen bei diesem Test z. B. die Verbindungen gemäß folgender Herstellungsbeispiele : 17 und 21.

## Patentansprüche

1. Azolyl-alkenone und -ole der Formel

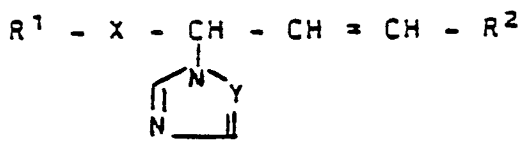

(I)

in welcher $R^1$ für die Gruppierung

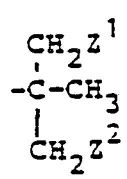

steht, in welcher

$Z^1$ und $Z^2$ gleich oder verschieden sind und für Wasserstoff, Halogen oder Alkyl mit 1 bis 4 Kohlenstoffatomen stehen, und ferner

$R^1$ für Phenyl steht, welches einfach oder mehrfach, gleichartig oder verschieden substituiert sein kann
durch Halogen, Alkyl mit 1 bis 4 Kohlenstoffatomen, Alkoxy und Alkylthio mit jeweils 1 bis 4 Kohlenstoffatomen, Alkyl- und Dialkylamino mit jeweils 1 bis 4 Kohlenstoffatomen in jedem Alkylteil, ferner Halogenalkyl, Halogenalkoxy und Halogenalkylthio mit jeweils 1 bis 2 Kohlenstoffatomen und 1 bis 5 gleichen oder verschiedenen Halogenatomen, Phenyl und/oder Phenoxy, wobei die beiden zuletzt genannten Reste ihrerseits durch Halogen und/oder Alkyl mit 1 bis 2 Kohlenstoffatomen substituiert sein können.

$R^2$ für geradkettiges oder verzweigtes Alkyl mit 1 bis 12 Kohlenstoffatomen steht, ferner für Phenyl steht, welches einfach oder mehrfach, gleichartig oder verschieden substituiert sein kann durch Halogen, Alkyl mit 1 bis 4 Kohlenstoffatomen, Alkoxy und Alkylthio mit jeweils 1 bis 4 Kohlenstoffatomen, Alkyl- und Dialkylamino mit jeweils 1 bis 4 Kohlenstoffatomen in jedem Alkylteil, ferner Halogenalkyl, Halogenalkoxy und Halogenalkylthio mit jeweils 1 bis 2 Kohlenstoffatomen und 1 bis 5 gleichen oder verschiedenen Halogenatomen, Phenyl und/oder Phenoxy, wobei die beiden zuletzt genannten Reste ihrerseits durch Halogen und/oder Alkyl mit 1 bis 2 Kohlenstoffatomen substituiert sein können, oder

$R^2$ für gegebenenfalls durch Halogen und/oder Alkyl mit 1 bis 4 Kohlenstoffatomen substituiertes Cycloalkyl mit 3 bis 7 Kohlenstoffatomen oder für gegebenenfalls durch Halogen und/oder Alkyl mit 1 bis 4 Kohlenstoffatomen substituiertes Cycloalkylalkyl mit 3 bis 7 Kohlenstoffatomen im Cycloalkylteil und 1 bis 2 Kohlenstoffatomen im Alkylteil steht,

X für die CO- oder CH(OH)-Gruppe steht und

Y für ein Stickstoffatom oder die CH-Gruppe steht,

sowie deren Säureadditions-Salze und Metallsalz-Komplexe.

2. Azolyl-alkenon der Formel

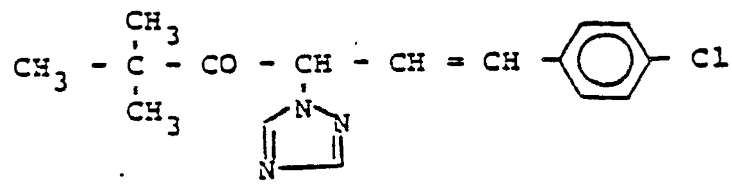

3. Azolyl-alkenon der Formel

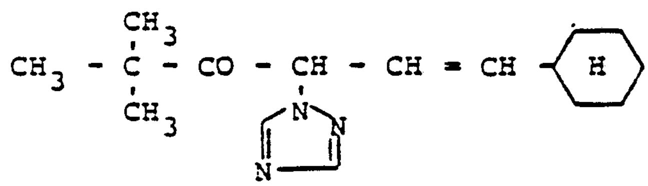

4. Azolyl-alkenol der Formel

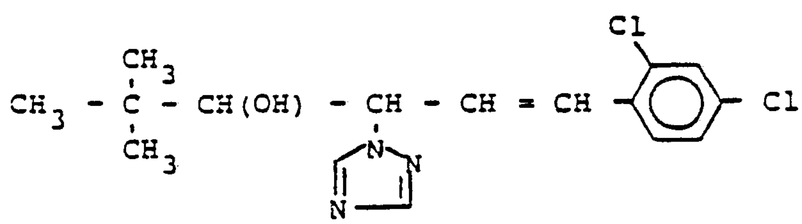

5. Verfahren zur Herstellung von Azolyl-alkenonen und -olen der Formel

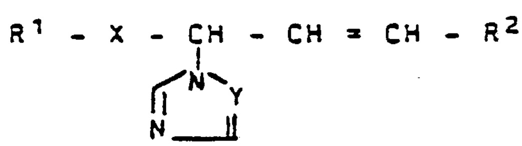

(I)

in welcher $R^1$ für die Gruppierung

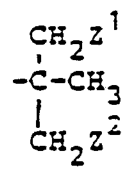

steht, in welcher

$Z^1$ und $Z^2$ gleich oder verschieden sind und für Wasserstoff, Halogen oder Alkyl mit 1 bis 4 Kohlenstoffatomen stehen, und ferner

$R^1$ für Phenyl steht, welches einfach oder mehrfach, gleichartig oder verschieden substituiert sein kann.
durch Halogen, Alkyl mit 1 bis 4 Kohlenstoffatomen, Alkoxy und Alkylthio mit jeweils 1 bis 4 Kohlenstoffatomen, Alkyl- und Dialkylamino mit jeweils 1 bis 4 Kohlenstoffatomen in jedem Alkylteil, ferner Halogenalkyl, Halogenalkoxy und Halogenalkylthio mit jeweils 1 bis 2 Kohlenstoffatomen und 1 bis 5 gleichen oder verschiedenen Halogenatomen, Phenyl und/oder Phenoxy, wobei die beiden zuletzt genannten Reste ihrerseits durch Halogen und/oder Alkyl mit 1 bis 2 Kohlenstoffatomen substituiert sein können,

$R^2$ für geradkettiges oder verzweigtes Alkyl mit 1 bis 12 Kohlenstoffatomen steht, ferner für Phenyl steht, welches einfach oder mehrfach, gleichartig oder verschieden substituiert sein kann durch Halogen, Alkyl mit 1 bis 4 Kohlenstoffatomen, Alkoxy und Alkylthio mit jeweils 1 bis 4 Kohlenstoffatomen, Alkyl- und Dialkylamino mit jeweils 1 bis 4 Kohlenstoffatomen in jedem Alkylteil, ferner Halogenalkyl, Halogenalkoxy und Halogenalkylthio mit jeweils 1 bis 2 Kohlenstoffatomen und 1 bis 5 gleichen oder verschiedenen Halogenatomen, Phenyl und/oder Phenoxy, wobei die beiden zuletzt genannten Reste ihrerseits durch Halogen und/oder Alkyl mit 1 bis 2 Kohlenstoffatomen substituiert sein können, oder

$R^2$ für gegebenenfalls durch Halogen und/oder Alkyl mit 1 bis 4 Kohlenstoffatomen substituiertes Cycloalkyl mit 3 bis 7 Kohlenstoffatomen oder für gegebenenfalls durch Halogen und/oder Alkyl mit 1 bis 4 Kohlenstoffatomen substituiertes Cycloalkylalkyl mit 3 bis 7 Kohlenstoffatomen im Cycloalkylteil und 1 bis 2 Kohlenstoffatomen im Alkylteil steht,

X für die CO- oder CH(OH)-Gruppe steht und

Y für ein Stickstoffatom oder die CH-Gruppe steht,

sowie deren Säureadditions-Salzen und Metallsalzkomplexen, dadurch gekennzeichnet, daß man Verbindungen der Formel

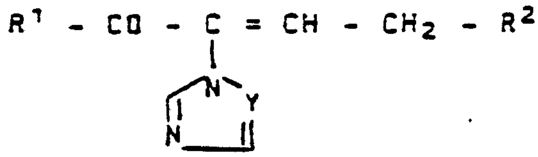

(II)

in welcher $R^1$, $R^2$ und Y die oben angegebene Bedeutung haben, in Gegenwart eines Verdünnungsmittels und gegebenenfalls in Gegenwart eines Katalysators bzw. in Gegenwart von Aluminiumoxid erhitzt und gegebenenfalls die dabei erhaltenen erfindungsgemäßen Azolylalkenone der Formel

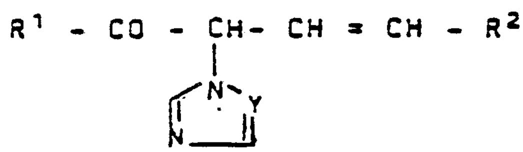

(Ia)

in welcher $R^1$, $R^2$ und Y die oben angegebene Bedeutung haben, in allgemein üblicher Weise reduziert und gegebenenfalls anschließend an die so erhaltenen Verbindungen der Formel (I) eine Säure oder ein Metallsalz addiert.

6. Pflanzenwachstumsregulierende und fungizide Mittel, gekennzeichnet durch einen Gehalt an mindestens einem (Azolyl-alkenon oder ol der Formel (I) bzw. einem Säureadditions-Salz oder Metallsalz-Komplex eines Azolyl-alkenons oder -ols der Formel (I).

7. Verfahren zur Regulierung des Pflanzenwachstums sowie zur Bekämpfung von Pilzen, dadurch gekennzeichnet, daß man Azolyl-alkenone oder -ole der Formel (I) bzw. deren Säureadditions-Salze oder Metallsalz-Komplexe auf die Pflanzen und/oder deren Lebensraum bzw. auf die Pilze oder deren Lebensraum einwirken läßt.

8. Verwendung von Azolyl-alkenonen und -olen der Formel (I) bzw. von Säureadditions-Salzen oder Metallsalz-Komplexen von Azolyl-alkenonen und -olen der Formel (I) zur Regulierung des Pflanzenwachstums bzw. zur Bekämpfung von Pilzen.

9. Verfahren zur Herstellung von pflanzenwachstumsregulierenden Mitteln bzw. von fungiziden Mitteln, dadurch gekennzeichnet, daß man Azolyl-alkenone oder -ole der Formel (I) bzw. Säureadditions-Salze oder Metallsalz-Komplexe von Azolyl-alkenonen oder -olen der Formel (I) mit Streckmitteln und/oder oberflächenaktiven Stoffen vermischt.

**Claims**

1. Azolyl-alkenones and -ols of the formula

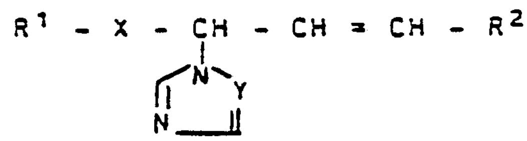

(I)

in which $R^1$ represents the grouping

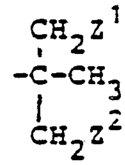

in which

$Z^1$ and $Z^2$ are identical or different and represent hydrogen, halogen or alkyl with 1 to 4 carbon atoms, and, furthermore,

$R^1$ represents phenyl which can be mono- or polysubstituted by identical or different substituents selected from halogen, alkyl with 1 to 4 carbon atoms, alkoxy and alkylthio each with 1 to 4 carbon atoms, alkyl- and dialkylamino each with 1 to 4 carbon atoms in each alkyl part, and also halogenoalkyl, halogenoalkoxy and halogenoalkylthio each with 1 to 2 carbon atoms and 1 to 5 identical or different halogen atoms, phenyl and/or phenoxy, it being possible for the two last-mentioned radicals in turn to be substituted by halogen and/or alkyl with 1 to 2 carbon atoms,

$R^2$ represents straight-chain or branched alkyl with 1 to 12 carbon atoms, and also phenyl which can be mono- or polysubstituted by identical or different substitutents selected from halogen, alkyl with 1 to 4 carbon atoms, alkoxy and alkylthio each with 1 to 4 carbon atoms, alkyl- and dialkylamino each with 1 to 4 carbon atoms in each alkyl part, and also halogenoalkyl, halogenoalkoxy and halogenoalkylthio each with 1 to 2 carbon atoms and 1 to 5 identical or different halogen atoms, phenyl and/or phenoxy, it being possible for the two last-mentioned radicals in turn to be substituted by halogen and/or alkyl with 1 to 2 carbon atoms, or

$R^2$ represents cycloalkyl which has 3 to 7 carbon atoms and is optionally substituted by halogen and/or alkyl with 1 to 4 carbon atoms or cycloalkylalkyl which has 3 to 7 carbon atoms in the cycloalkyl part and 1 to 2 carbon atoms in the alkyl part and is optionally substituted by halgoen and/or alkyl with 1 to 4 carbon atoms,

X represents the CO or CH(OH) group and

Y represents a nitrogen atom or the CH group,

and acid addition salts and metal salt complexes thereof.

2. An azolyl-alkenone of the formula

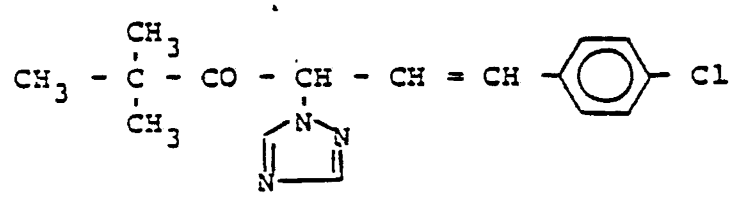

3. An azolyl-alkenone of the formula

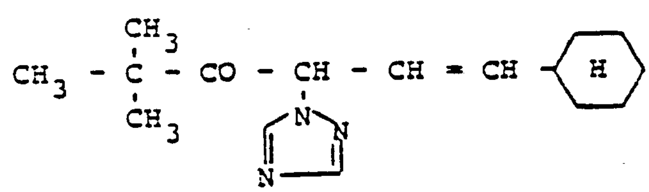

4. An azolyl-alkanol of the formula

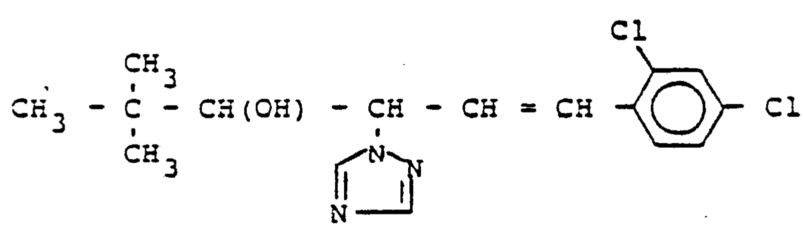

5. Process for the preparation of azolyl-alkenones and -ols of the formula

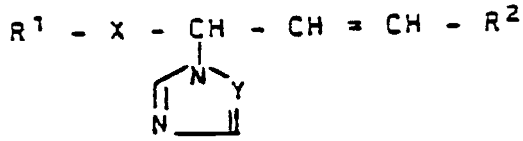

(I)

in which $R^1$ represents the grouping

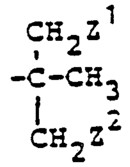

in which

$Z^1$ and $Z^2$ are identical or different and represent hydrogen, halogen or alkyl with 1 to 4 carbon atoms, and, furthermore,

$R^1$ represents phenyl which can be mono- or polysubstituted by identical or different substituents selected from halogen, alkyl with 1 to 4 carbon atoms, alkoxy and alkylthio each with 1 to 4 carbon atoms, alkyl- and dialkylamino each with 1 to 4 carbon atoms in each alkyl part, and also halogenoalkyl, halogenoalkoxy and halogenoalkylthio each with 1 to 2carbon atoms and 1 to 5 identical or different halogen atoms, phenyl and/or phenoxy, it being possible for the two last-mentioned radicals in turn to be substituted by halogen and/or alkyl with 1 to 2 carbon atoms,

$R^2$ represents straight-chain or branched alkyl with 1 to 12 carbon atoms, and also phenyl which can be mono- or polysubstituted by identical or different substituents selected from halogen, alkyl with 1 to 4 carbon atoms, alkoxy and alkylthio each with 1 to 4 carbon atoms, alkyl- and dialkylamino each with 1 to 4 carbon atoms in each alkyl part, and also halogenoalkyl, halogenoalkoxy and halogenoalkylthio each with 1 to 2 carbon atoms and 1 to 5 identical or different halogen atoms, phenyl and/or phenoxy, it being possible for the two last-mentioned radicals in turn to be substituted by halogen and/or alkyl with 1 to 2 carbon atoms, or

$R^2$ represents cycloalkyl which has 3 to 7 carbon atoms and is optionally substituted by halogen and/or alkyl with 1 to 4 carbon atoms or cycloalkylalkyl which has 3 to 7 carbon atoms in the cycloalkyl part and 1 to 2 carbon atoms in the alkyl part and is optionally substituted by halogen and/or alkyl with 1 to 4 carbon atoms,

X represents the CO or CH(OH) group and

Y represents a nitrogen atom or the CH group,

and acid addition salts and metal salt complexes thereof, characterised in that compounds of the formula

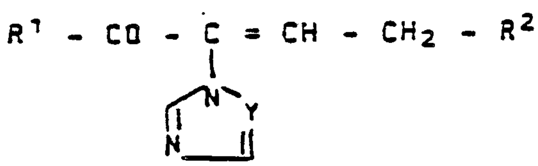

(II)

in which $R^1$, $R^2$ and Y have the meaning indicated above, are heated in the presence of a diluent and, if appropriate, in the presence of a catalyst or in the presence of aluminium oxide and, if desired, the azolyl alkenones according to the invention which are thereby obtained and have the formula

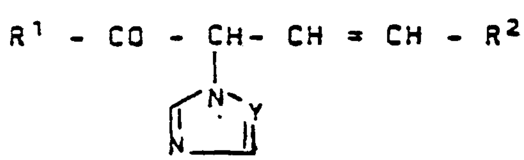

(Ia)

in which $R^1$, $R^2$ and Y have the meaning indicated above, are reduced in a generally customary manner and, if desired, an acid or a metal salt is then added on to the compounds of the formula (I) thus obtained.

6. Plant-growth-regulating and fungicidal agents, characterised in that they contain at least one azolyl-alkenone or -ol of the formula (I) or at least one acid addition salt or metal salt complex of an azolyl-alkenone or -ol of the formula (I).

7. Method of regulating plant growth and of combating fungi, characterised in that azolyl-alkenones or -ols of the formula (I) or acid addition salts or metal salt complexes thereof are allowed to act on the plants and/or their environment or on the fungi or their environment.

8. Use of azolyl-alkenones and -ols of the formula (I) or of acid addition salts or metal salt complexes of azolyl-alkenones and -ols of the formula (I) for regulating plant growth or for combating fungi.

9. Process for the preparation of plant-growth-regulating agents or fungicidal agents, characterised in that azolyl-alkenones or -ols of the formula (I) or acid addition salts or metal salt complexes of azolyl-alkenones or -ols of the formula (I) are mixed with extenders and/or surface-active substances.

**Revendications**

1. Azolyl-alcénones et -ols de formule

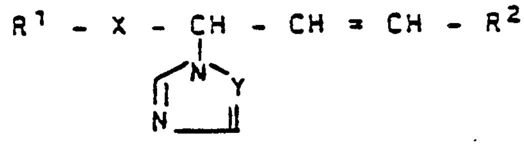

(I)

dans laquelle $R^1$ représente le groupement

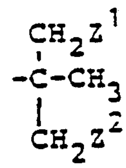

dans lequel

$Z^1$ et $Z^2$ sont égaux ou différents et représentent l'hydrogène, un halogène ou un groupe alkyle ayant 1 à 4 atomes de carbone, et en outre

$R^1$ représente un groupe phényle qui peut porter un ou plusieurs substituants égaux ou différents choisis entre

des halogènes, des restes alkyle ayant 1 à 4 atomes de carbone, alkoxy et alkylthio ayant chacun 1 à 4 atomes de carbone, alkyl- et dialkylamino ayant chacun 1 à 4 atomes de carbone dans chaque partie alkyle, en outre des restes halogénalkyle, halogénalkoxy et halogénalkylthio ayant chacun 1 ou 2 atomes de carbone et 1 à 5 atomes d'halogènes égaux ou différents, le reste phényle et/ou le reste phénoxy, les deux restes mentionnés en dernier lieu pouvant être substitués quant à eux par un halogène et/ou un groupe alkyl ayant 1 ou 2 atomes de carbone

$R^2$ désigne un groupe alkyle à chaîne droite ou ramifiée ayant 1 à 12 atomes de carbone, en outre un groupe phényle qui peut porter un ou plusieurs substituants égaux ou différents choisis entre des halogènes, des restes alkyle ayant 1 à 4 atomes de carbone, alkoxy et alkylthio ayant chacun 1 à 4 atomes de carbone, alkyl- et dialkylamino ayant chacun 1 à 4 atomes de carbone dans chaque partie alkyle, en outre des restes halogénalkyle, halogénalkoxy et halogénalkylthio ayant chacun 1 ou 2 atomes de carbone et 1 à 5 atomes d'halogènes égaux ou différents, un reste phényle et/ou un reste phénoxy, les deux restes mentionnés en dernier lieu pouvant être substitués quant à eux par des halogènes et/ou des groupes alkyle ayant 1 ou 2 atomes de carbone, ou bien

$R^2$ est un groupe cycloalkyle de 3 à 7 atomes de carbone éventuellement substitué par un halogène et/ou un substituant alkyle ayant 1 à 4 atomes de carbone, ou un reste cycloalkylalkyle éventuellement substitué par un halogène et/ou un substituant alkyle ayant 1 à 4 atomes de carbone, avec 3 à 7 atomes de carbone dans la partie cycloalkyle et 1 ou 2 atomes de carbone dans la partie alkyle,

X est un groupe CO ou CH(OH) et

Y est un atome d'azote ou le groupe CH,

ainsi que leurs sels d'addition d'acides et leurs complexes de sels métalliques.

2. Azolyl-alcénone de formule

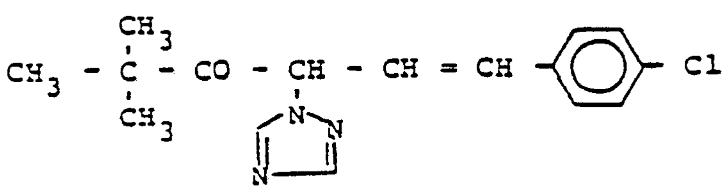

3. Azolyl-alcénone de formule

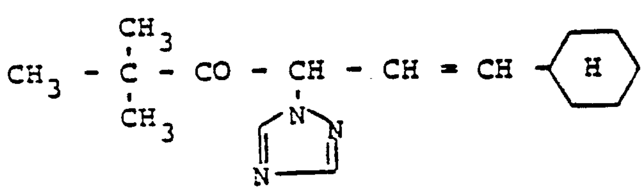

4. Azolyl-alcénol de formule

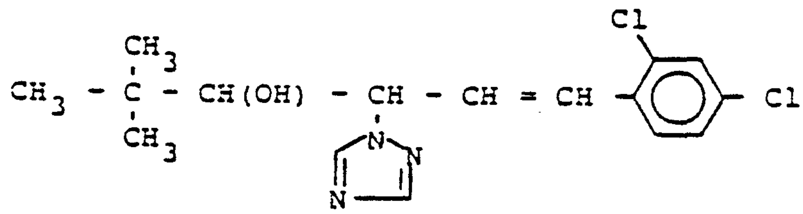

5. Procédé de production d'azolyl-alcénones et -ols de formule

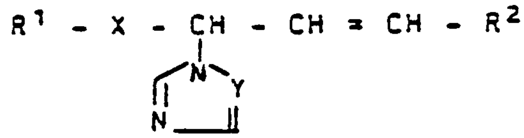

dans laquelle $R^1$ représente le groupe

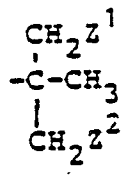

(I)

dans lequel

$Z^1$ et $Z^2$ sont égaux ou différents et représentent l'hydrogène, un halogène ou un groupe alkyle ayant 1 à 4 atomes de carbone, et en outre

$R^1$ représente un groupe phényle qui peut porter un ou plusieurs substituants égaux ou différents choisis entre

des halogènes, des restes alkyle ayant 1 à 4 atomes de carbone, alkoxy et alkylthio ayant chacun 1 à 4 atomes de carbone, alkyl- et dialkylamino ayant chacun 1 à 4 atomes de carbone dans chaque partie alkyle, en outre des restes halogénalkyle, halogénalkoxy et halogénalkylthio ayant chacun 1 ou 2 atomes de carbone et 1 à 5 atomes d'halogènes égaux ou différents, le reste phényle et/ou le reste phénoxy, les deux restes mentionnés en dernier lieu pouvant être substitués quant à eux par un halogène et/ou un groupe alkyle ayant 1 ou 2 atomes de carbone

$R^2$ désigne un groupe alkyle à chaîne droite ou ramifiée ayant 1 à 12 atomes de carbone, en outre un groupe phényle qui peut porter un ou plusieurs substituants égaux ou différents choisis entre des halogènes, des restes alkyle ayant 1 à 4 atomes de carbone, alkoxy et alkylthio ayant chacun 1 à 4 atomes de carbone, alkyl- et dialkylamino ayant chacun 1 à 4 atomes de carbone dans chaque partie alkyle, en outre des restes halogénalkyle, halogénalkoxy et halogénalkylthio ayant chacun 1 ou 2 atomes de carbone et 1 à 5 atomes d'halogènes égaux ou différents, un reste phényle et/ou un reste phénoxy, les deux restes mentionnés en dernier lieu pouvant être substitués quant à eux par des halogènes et/ou des groupes alkyle ayant 1 ou 2 atomes de carbone, ou bien

$R^2$ est un groupe cycloalkyle de 3 à 7 atomes de carbone éventuellement substitué par un halogène et/ou un substituant alkyle ayant 1 à 4 atomes de carbone, ou un reste cycloalkylalkyle éventuellement substitué par un halogène et/ou un substituant alkyle ayant 1 à 4 atomes de carbone, avec 3 à 7 atomes de carbone dans la partie cycloalkyle et 1 ou 2 atomes de carbone dans la partie alkyle,

X est un groupe CO ou CH(OH)

Y est un atome d'azote ou le groupe CH,

ainsi que de leurs sels d'addition d'acides et de leurs complexes de sels métalliques, caractérisé en ce qu'on chauffe des composés de formule

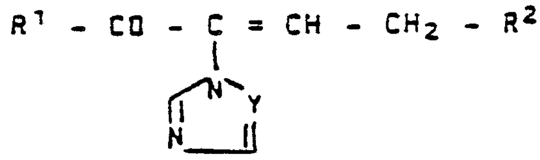

(II)

dans laquelle $R^1$, $R^2$ et Y ont la définition indiquée ci-dessus, en présence d'un diluant et le cas échéant en présence d'un catalyseur ou respectivement en présence d'oxyde d'aluminium et, le cas échéant, on réduit d'une manière généralement classique les azolylalcénones conformes à l'invention, ainsi obtenues, de formule

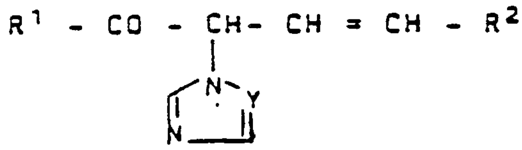

(Ia)

dans laquelle $R^1$, $R^2$ et Y ont la définition indiquée ci-dessus, puis on additionne éventuellement sur les composés de formule (I) ainsi obtenus un acide ou un sel métallique.

6. Compositions influençant la croissance des végétaux et à action fongicide, caractérisées par une teneur en au moins une azolylalcénone ou au moins un azolylalcénol de formule (I) ou d'un sel d'addition d'acide ou d'un complexe de sel métallique d'une azolylalcénone ou d'un azolylalcénol de formule (I).

7. Procédé pour influencer la croissance des végétaux ainsi que pour combattre des champignons, caractérisé en ce qu'on fait agir des azolylalcénones ou des azolylalcénols de formule (I) ou leurs sels d'addition d'acides ou leurs complexes de sels métalliques sur les plantes et/ou sur leur milieu ou respectivement sur les champignons ou sur leur milieu.

8. Utilisation d'azolylalcénones et -ols, de formule (I) ou de sels d'addition d'acides ou de complexes de sels métalliques d'azolylalcénones et -ols de formule (I) pour influencer la croissance des végétaux ou pour combattre des champignons.

9. Procédé de préparation de compositions influençant la croissance des végétaux et de compositions fongicides, caractérisé en ce qu'on mélange des alzolylalcénones ou -ols de formule (I) ou des sels d'addition d'acides ou des complexes de sels métalliques d'azolylalcénones ou -ols de formule (I) avec des diluants et/ou des agents tensio-actifs.